**Europäisches Patentamt**

**European Patent Office**

**Office européen des brevets**

(19)

(11) Veröffentlichungsnummer: **0 250 955**
**A1**

(12) # EUROPÄISCHE PATENTANMELDUNG

(21) Anmeldenummer: **87108349.9**

(22) Anmeldetag: **10.06.87**

(51) Int. Cl.4: **C07C 143/76 , C07C 143/70**

(30) Priorität: **20.06.86 DE 3620667**

(43) Veröffentlichungstag der Anmeldung:
**07.01.88 Patentblatt 88/01**

(84) Benannte Vertragsstaaten:
**AT BE CH DE ES FR GB IT LI NL SE**

(71) Anmelder: **Kali-Chemie Aktiengesellschaft**
**Postfach 220 Hans-Böckler-Allee 20**
**D-3000 Hannover 1(DE)**

(72) Erfinder: **Mechthold, Gerhard**
**Rotekreuzstrasse 11**
**D-3000 Hannover 61(DE)**
Erfinder: **Epkens, Georg**
**Kronenstrasse 27**
**D-3000 Hannover 1(DE)**
Erfinder: **Heinemann, Henning**
**Im Bruche 14A**
**D-3160 Lehrte-Aligse(DE)**

(74) Vertreter: **Lauer, Dieter, Dr.**
**c/o Kali-Chemie Aktiengesellschaft Postfach 220**
**D-3000 Hannover 1(DE)**

(54) **Verfahren zur Herstellung von 2-Aminoäthansulfonamid.**

(57) 2-(Trifluoracetylamino)-äthansulfonsäurechlorid wird zunächst mit Ammoniak zum entsprechenden Amid umgesetzt, das dann weiter mit Ammoniak zu 2-Aminoäthansulfonamid umgesetzt wird.

**EP 0 250 955 A1**

## Verfahren zur Herstellung von 2-Aminoäthansulfonamid

Die vorliegende Erfindung betrifft die Herstellung von 2-Aminoäthansulfonamid sowie dessen Trifluoracetyl-Derivat als neues Zwischenprodukt.

2-Aminoäthansulfonamid (im folgenden Taurinamid genannt) wird üblicherweise aus Taurin in einem Vielstufenverfahren hergestellt, bei dem zunächst das N-terminale Ende der β-Aminoäthansulfonsäure geschützt wird. Nach Aktivierung der Sulfonsäuregruppe wird dann mittels NH₃-Einwirkung das geschützte Taurinamid erzeugt.

Die üblicherweise angewandten Schutzgruppen wie Carbobenzoxy-, Fmoc-, Phthaloyl-Gruppe u.a. sind für einen technischen Einsatz ungeeignet. Carbobenzoxy aufgrund der bekannten Nachteile bei der Abspaltung (Hydrierung bzw. HBr/Eisessig), Fmoc wegen der hohen Kosten, Phthaloyl wegen der bekannten Cancerogenität des für die Abspaltung notwendigen Hydrazins.

Aufgabe der Erfindung ist es daher, ein verbessertes Verfahren zur Herstellung von Taurinamid zur Verfügung zu stellen.

Das erfindungsgemäße Verfahren ist durch das in den Patentansprüchen beschriebene Verfahren charakterisiert.

Es ist insbesondere dadurch gekennzeichnet, daß man

a) 2-(Trifluoracetylamino)-äthansulfonsäurechlorid mit Ammoniak als Amin umsetzt und

b) das als Zwischenprodukt entstehende 2-(Trifluoracetylamino)-äthansulfonamid, entweder direkt oder nach seiner Isolierung und gegebenenfalls Reinigung, mit weiterem Ammoniak zum 2-Aminoäthansulfonamid umsetzt.

Das als Ausgangsverbindung einzusetzende 2-(Trifluoracetylamino)-äthansulfonsäurechlorid (TFE-Taurinchlorid) ist als solches bereits bekannt und wird aus Taurin durch Umsetzung nacheinander mit einer geeigneten Trifluoracetyl-Verbindung und einem Chlorierungsmittel wie beispielsweise Sulfurylchlorid, Thionylchlorid, etc. hergestellt.

Vorzugsweise wird dazu ein Alkali-oder Ammoniumsalz der Aminoäthansulfonsäure, insbesondere das Kalium, Natrium oder Trialkylammoniumsalz, mit geeigneten Trifluoressigsäurederivaten wie Trifluoressigsäureanhydrid, -chlorid, -ester, in Methanol umgesetzt, worauf das entstandene Trifluoracetyl-aminoäthansulfonat mit Phosphorpentachlorid oder Sulfurylchlorid bzw. Thionylchlorid in einem inerten Lösungsmittel wie Dichlormethan bzw. Toluol u.a. umgesetzt wird.

Das TFE-Taurinchlorid kann erfindungsgemäß nach zwei verschiedenen Varianten umgesetzt werden, nämlich entweder

A) in einer Eintopfreaktion unter Beibehaltung des Lösungsmittelsystems oder

B) in einer zweistufigen Reaktion unter Wechsel des Lösungsmittels.

Als Lösungsmittel - im Falle A) für beide Stufen bzw. im Fall B) für die erste Stufe - könne inerte Lösungsmittel wie beispielsweise Äther, Amide, Halogenkohlenwasserstoffe oder deren Gemische eingesetzt werden. Aus diesen Gruppen können insbesondere Diäthyläther, Tetrahydrofuran, Dimethylformamid, Methylenchlorid verwendet werden.

In einer anderen Variante kann als Lösungsmittel - im Fall A) für beide Stufen, im Fall B) für die zweite Stufe - Wasser oder Wasser im Gemisch mit wasserverträglichen Lösungsmitteln aus der o.a. Gruppe eingesetzt werden.

Die Umsetzung erfolgt dabei vorzugsweise durch Einleitung von NH₃ in das Gemisch aus Lösungsmittel und Ausgangsverbindung bei verminderter Temperatur, vorzugsweise bei - 5 bis - 40 °C. NH₃ wird dabei im Überschuß zugegeben und agiert sowohl als Amidierungsreagenz als auch als säurebindendes Agenz. Die Reaktionszeit variiert mit der gewählten Temperatur und kann in üblicher Weise optimiert werden, z.B. durch chromatographische Verfolgung des Umsatzgrades.

In einer Variante der Reaktionsführung kann die Ausgangsverbindung auch in eine vorgelegte, gekühlte Lösung von NH₃ im Lösungsmittel eingegeben werden. Dabei ist auf eine effektive Abführung der Reaktionswärme zu achten.

In Variante A) (Eintopfreaktion) wird im Anschluß an die Amidierung des Reaktionsgemisches auf Raumtemperatur, vorzugsweise auf eine erhöhte Temperatur im Bereich von + 30 bis + 60 °C erwärmt, wobei - sofern nicht von vornherein ein entsprechender NH₃-Überschuß dosiert worden war - weiteres NH₃ eingeleitet wird. Die Abspaltung der N-terminalen Schutzgruppe ist je nach Temperatur nach etwa 1 Stunde Erwärmung abgeschlossen. Aus dem Reaktionsgemisch kann Taurinamid nach an sich bekannten Methoden wie chromatographische Verfahren, Fällung (z.B. als Hydrochlorid etc.) gewonnen werden.

In Variante B) wird im Anschluß an die Amidierung das Lösungsmittel abgezogen, der Rückstand mit wässrigem Ammoniak versetzt und - wie vorstehend beschrieben - weiter verfahren.

Im Verlauf der Umsetzung tritt 2-(Trifluoracetylamino)-äthansulfonamid (TFE-Taurinamid) als Zwischenprodukt auf, das durch an sich bekannte Verfahrensschritte, wie z.B. chromatographische Verfahren, isoliert werden kann.

Diese Verbindung ist neu. Sie bildet farblose Kristalle mit einem Schmelzpunkt von 127 bis 128 °C und weist folgendes NMR-Spektrum auf:

$^{13}$C-NMR (DMSO-d$_6$, Me$_4$Si):δ 115,9 (q, CF$_3$, $^1J_{C,F}$ 288), 156,5 (q, C = O, $^2J_{C,F}$ 37), 52,6 (t, CH$_2$, $^1J_{C,H}$ 138), 34,8 (t, CH$_2$, $^1J_{C,H}$ 144).

Sie kann beispielsweise - in einer Retrosynthese - auch durch Umsetzung von Taurinamid mit Trifluoracetyl-liefernden Ausgangsverbindungen der obengenannten Art erhalten werden.

Es ist zwar aus der DE-OS 28 25 289 bereits bekannt, TFE-Taurinchlorid mit Aminoglykosiden in einem zweistufigen verfahren umzusetzen. Dabei entsteht ein TFE-geschütztes Taurin-Aminoglykosid, das auch isoliert werden kann. Dieser Veröffentlichung ist allerdings kein Hinweis entnehmbar, daß auch im Falle des Einsatzes von NH$_3$ als Amin ein isolierbares Zwischenprodukt gebildet wird. Unter den Bedingungen der Vorveröffentlichung konnte bei einer analogen Nacharbeitung mit NH$_3$ als Amin ein solches Zwischenprodukt auch nicht isoliert werden, was z.B. an der anderen Verfahrensführung (Temperatur und/oder Triäthylamin-Zusatz etc.) liegen mag.

Das TFE-Taurinamid kann zur vorteilhaften Variation oder Abänderung des Verfahrens verwendet werden.

Es bietet sich an, TFE-Taurinamid - sei es nach Isolierung oder in situ - als Ausgangsprodukt für TFE-Taurinamid-Derivate einzusetzen.

Die folgenden Beispiele sollen die Erfindung weiter erläutern, ohne ihren Umfang zu begrenzen.

Beispiele

Beispiel 1

1. Stufe Trifluoracetyltaurin

50 g Taurin werden in ein Gemisch aus 200 ml Methanol und 88 g Triethylamin eingetragen. Anschließend werden 58 g Trifluoracetylchlorid unter Rühren eingeleitet. Dabei erfolgt Erwärmung bis zum Sieden des Methanols. Nach Beendigung der Zugabe wird der Ansatz noch 1 Stunde am Sieden gehalten, danach wird das Methanol abdestilliert.

2. Stufe Trifluoracetyltaurinchlorid

Der zähflüssige Rückstand aus "Stufe 1" wird in 250 ml Methylenchlorid aufgenommen und zum Sieden erhitzt. Anschließend werden 35 ml Thionylchlorid langsam zudosiert. Danach wird noch ca. 1 Stunde am Rückfluß erhitzt, anschließend werden das Lösemittel und überschüssiges Thionylchlorid abdestilliert.

3. Stufe Trifluoracetyltaurinamid

Der Rückstand aus "Stufe 2" wird in 900 ml Methylenchlorid aufgenommen. Bei -10 °C wird ca. 3 Stunden lang Ammoniakgas eingeleitet. Danach wird der Ansatz mehrere Stunden lang bei -10 °C gerührt, anschließend läßt man langsam auf Zimmertemperatur erwärmen und über Nacht stehen.

Das Vorliegen von Trifluoracetyltaurinamid im Reaktionsgemisch konnte mittels HPLC (reversed phase-Verfahren an Bondapak C 18 als Säulenmaterial und Acetonitril/Wasser als mobile Phase) belegt werden. Als Referenzmaterial diente ein gemäß Beispiel 2 hergestelltes Trifluoracetyltaurinamid.

4. Stufe Taurinamid - HCl

Variante A

Die Reaktionslösung aus "Stufe 3" wird zum Sieden erhitzt und 2 Stunden am Rückfluß gehalten. Danach wird das Lösemittel nebst überschüssigem Ammoniak abdestilliert, der Rückstand in ca. 200 ml Waser aufgenommen.

Variante B

Aus der Reaktionslösung aus "Stufe 3" wird das Lösemittel abdestilliert. Der Rückstand wird in 290 ml 25 %iger wässriger Ammoniaklösung aufgenommen, danach wird die Lösung 2 stunden bei 40 bis 50 °C gehalten, anschließend der überschüssige Ammoniak abdestilliert. Im Rückstand verbleibt eine wässrige Lösung, die identisch ist mit der aus "Variante A".

Die Aufarbitung der beiden Lösungen erfolgt auf die gleiche Weise nach bekannten chromatographischen Methoden.

Beispiel 2

Herstellung von Trifluoracetyltaurinamid

Aus 100 g Taurinamid-Hydrochlorid wurde durch Behandlung mit stark saurem Ionenaustauscher und Elution mittels Ammoniak die Base freigesetzt. Das eingeengte Eluat wurde in Methanol aufgenommen. In die klare Lösung wird unter Sieden tropfenweise die äquivalente Menge Trifluoressigsäuremethylester zugesetzt. Es wurden zur Nachreaktion 3 Stunden in der Hitze, dann 2 Stunden unter Eiskühlung weitergerührt. Das ausgefallene Trifluoracetyltaurinamid wurde abgesaugt, mit Methanol gewaschen und getrocknet. Ausbeute: 70 g. Schmelzpunkt und NMR-Spektrum wie in der Beschreibung angegeben.

## Ansprüche

1) Verfahren zur Herstellung von ω-Aminoalkansulfonamiden durch Umsetzung von ω-(Trifluoracetylamino)-äthansulfonsäurechlorid mit einem Amin und hydrolytischer Abspaltung der N-Schutzgruppe mittels Ammoniak, dadurch gekennzeichnet, daß man

a) 2-(Trifluoracetylamino)-äthansulfonsäurechlorid mit Ammoniak als Amin umsetzt und

b) das als Zwischenprodukt entstehende 2-(Trifluoracetylamino)-äthansulfonamid, entweder direkt oder nach seiner Isolierung und gegebenenfalls Reinigung, mit weiterem Ammoniak zum 2-Aminoäthansulfonamid umsetzt.

2) Verfahren nach Anspruch 1, dadurch gekennzeichnet, daß man in Stufe a) und b) im gleichen Lösungsmittel arbeitet.

3) Verfahren nach Anspruch 1, dadurch gekennzeichnet, daß man in Stufe a) und b) in verschiedenen Lösungsmitteln arbeitet.

4) Verfahren nach einem der Ansprüche 1 bis 3, dadurch gekennzeichnet, daß man mindestens in Stufe a) in einem inerten Lösungsmittel, vorzugsweise in Halogenkohlenwasserstoffen wie Methylenchlorid, arbeitet.

5) Verfahren nach einem der Ansprüche 1 bis 3, dadurch gekennzeichnet, daß man mindestens in Stufe b) in Wasser oder wässrigen Lösungen als Lösungsmittel arbeitet.

6) Verfahren nach einem der vorhergehenden Ansprüche, dadurch gekennzeichnet, daß man in Stufe a) bei verminderter Temperatur, insbesondere bei Temperaturen von -40 bis -5 °C und in Stufe b) bei erhöhter Temperatur, insbesondere bei +30 bis +60°C arbeitet.

7) 2-(Trifluoracetylamino)-äthansulfonamid.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | FR-A-2 393 811 (MEIJI SEIRA KAISHA) <br> * Ansprüche; Beispiel 1 * | 1 | C 07 C 143/76 <br> C 07 C 143/70 |
| | --- | | |
| Y | US-A-2 184 279 (W.G. CHRISTIANSEN) <br> * Ansprüche * | 1 | |
| | --- | | |
| Y | DE-A-1 812 937 (NAITO, SHUN-ICHI) <br> * Ansprüche * | 1 | |
| | ----- | | |

| RECHERCHIERTE SACHGEBIETE (Int. Cl.4) |
|---|
| C 07 C 143/00 |

Der vorliegende Recherchenbericht wurde für alle Patentansprüche erstellt.

| Recherchenort | Abschlußdatum der Recherche | Prüfer |
|---|---|---|
| DEN HAAG | 29-09-1987 | MOREAU J.M. |